(19) Europäisches Patentamt
European Patent Office
Office européen des brevets

(11) Veröffentlichungsnummer: **0 257 605**
**A1**

(12) # EUROPÄISCHE PATENTANMELDUNG

(21) Anmeldenummer: **87112241.2**

(22) Anmeldetag: **24.08.87**

(51) Int. Cl.⁴: **C07D 239/34 , A01N 47/34**

(30) Priorität: **26.08.86 CH 3422/86**
**23.07.87 CH 2795/87**

(43) Veröffentlichungstag der Anmeldung:
**02.03.88 Patentblatt 88/09**

(84) Benannte Vertragsstaaten:
**AT BE CH DE ES FR GB IT LI NL**

(71) Anmelder: **CIBA-GEIGY AG**
**Klybeckstrasse 141**
**CH-4002 Basel(CH)**

(72) Erfinder: **Sturm, Elmar, Dr.**
**Klusstrasse 66**
**CH-4147 Aesch(CH)**
Erfinder: **Lang, Robert Werner, Dr.**
**Hagenbachweg 10**
**CH-4133 Pratteln(CH)**
Erfinder: **Kristiansen, Odd, Dr.**
**Delligrabenstrasse 7**
**CH-4313 Möhlin(CH)**

(74) Vertreter: **Zumstein, Fritz, Dr. et al**
**Bräuhausstrasse 4**
**D-8000 München 2(DE)**

(54) **Substituierte Benzoylphenylharnstoffe.**

(57) Neue substituierte N-Benzoyl-N'-4-(5-phenylpyrimid-2-yloxy)-phenyl-harnstoffe der Formel

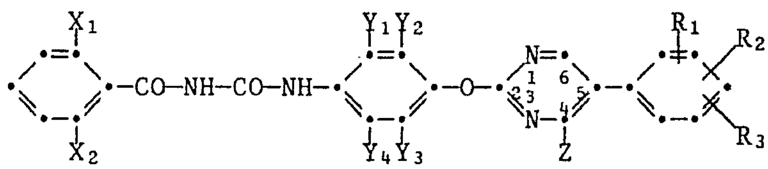

worin
$X_1$ Wasserstoff, Halogen, $C_1$-$C_3$-Alkoxy oder $C_1$-$C_3$-Alkylthio;
$X_2$ Halogen, Methyl, $C_1$-$C_3$-Alkoxy oder $C_1$-$C_3$-Alkylthio;
$Y_1$, $Y_2$, $Y_3$ und $Y_4$ unabhängig voneinander Wasserstoff, Halogen, Methyl, Trifluormethyl oder Methoxy;
Z Methyl, Halogenmethyl mit 1 bis 3 Halogenatomen oder Pentafluoräthyl;
$R_1$, $R_2$ und $R_3$ unabhängig voneinander Wasserstoff, Halogen, $C_1$-$C_3$-Alkyl, Trifluormethyl oder $C_1$-$C_3$-Alkoxt bedeuten;
Verfahren und Zwischenprodukte zur Herstellung dieser Verbindungen sowie diese enthaltende Mittel zur Verwendung in der Schädlingsbekämpfung, insbesondere zur Bekämpfung von Pflanzen und Tiere befallenden Vertretern der Ordnung Acarina und Insekten. Die neuen Verbindungen weisen speziell hohe Wirksamkeit gegen pflanzenschädigende Milben auf.

EP 0 257 605 A1

## Substituierte Benzoylphenylharnstoffe

Die vorliegende Erfindung betrifft neue substituierte N-Benzoyl-N'-4-(5-phenylpyrimidin-2-yloxy)-phenylharnstoffe, Verfahren und Zwischenprodukte zu ihrer Herstellung und ihre Verwendung in der Schädlingsbekämpfung.

Diese erfindungsgemässen Verbindungen haben die Formel I

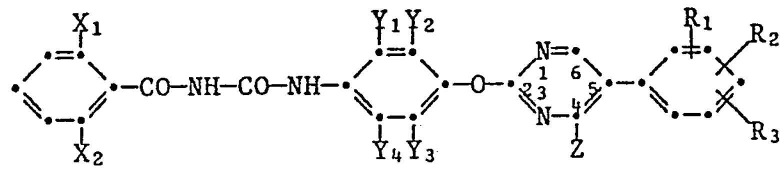

worin

$X_1$ Wasserstoff, Halogen, $C_1$-$C_3$-Alkoxy oder $C_1$-$C_3$-Alkylthio;

$X_2$ Halogen, Methyl, $C_1$-$C_3$-Alkoxy oder $C_1$-$C_3$-Alkylthio;

$Y_1$, $Y_2$, $Y_3$ und $Y_4$ unabhängig voneinander Wasserstoff, Halogen, Methyl, Trifluormethyl oder Methoxy;

Z Methyl, Halogenmethyl mit 1 bis 3 Halogenatomen oder Pentafluoräthyl;

$R_1$, $R_2$ und $R_3$ unabhängig voneinander Wasserstoff, Halogen, $C_1$-$C_3$-Alkyl, Trifluormethyl oder $C_1$-$C_3$-Alkoxy bedeuten.

Bevorzugt sind Verbindungen der Formel I, die dadurch gekennzeichnet sind, dass

$X_1$ Wasserstoff, Halogen, $C_1$-$C_3$-Alkoxy oder $C_1$-$C_3$-Alkylthio;

$X_2$ Halogen, Methyl, $C_1$-$C_3$-Alkoxy oder $C_1$-$C_3$-Alkylthio;

$Y_1$, $Y_2$, $Y_3$ und $Y_4$ unabhängig voneinander Wasserstoff, Halogen, Methyl, Trifluormethyl oder Methoxy;

Z Methyl oder Halogenmethyl mit 1 bis 3 Halogenatomen;

$R_1$, $R_2$ und $R_3$ unabhängig voneinander Wasserstoff, Halogen, $C_1$-$C_3$-Alkyl, Trifluormethyl oder $C_1$-$C_3$-Alkoxy bedeuten.

Wegen ihrer Wirkung als Schädlingsbekämpfungsmittel bevorzugt sind solche Verbindungen der Formel I, die dadurch gekennzeichnet sind, dass

$X_1$ Wasserstoff, Halogen oder Methoxy;

$X_2$ Halogen oder Methoxy;

$Y_1$ und $Y_4$ Wasserstoff;

$Y_2$ und $Y_3$ unabhängig voneinander Wasserstoff, Fluor, Chlor, Methyl oder Trifluormethyl;

Z Methyl, Dichlormethyl, Trichlormethyl, Monofluormethyl, Difluormethyl, Trifluormethyl, Difluorchlormethyl oder Pentafluoräthyl;

$R_1$, $R_2$ und $R_3$ unabhängig voneinander Wasserstoff, Fluor, Chlor, Brom, Methyl, Aethyl, i-Propyl, Trifluormethyl, Methoxy oder Aethoxy bedeuten.

Hervorzuheben sind ferner solche Verbindungen der Formel I, worin $X_1$ Fluor, Chlor oder Methoxy und

$X_2$ Fluor oder Chlor

bedeuten, und solche, worin

$Y_1$ und $Y_4$ Wasserstoff und

$Y_2$ und $Y_3$ Wasserstoff, Fluor oder Chlor bedeuten.

Von besonderem biologischen Interesse sind insbesondere diejenigen Verbindungen der Formel I, worin Z Trifluormethyl, Difluorchlormethyl oder auch Pentafluoräthyl bedeutet; diejenigen, worin

$R_1$ Wasserstoff,

$R_2$ Wasserstoff, 2-Fluor oder 2-Chlor und

$R_3$ Wasserstoff, 4-Fluor oder 4-Chlor bedeuten; sowie diejenigen, worin $X_1$ und $X_2$ Fluor und $R_1$, $R_2$ und $R_3$ Wasserstoff bedeuten; sowie diejenigen, worin $Y_1$ Methyl bedeutet.

Beispiele für Alkyl-Gruppen in der Definition $C_1$-$C_3$-Alkyl, -Alkoxy oder -Alkylthio gemäss der Erfindung sind Methyl, Aethyl, n-Propyl und i-Propyl. Im Rahmen der Erfindung ist unter Halogen Fluor, Chlor, Brom und Jod, vorzugsweise Fluor und Chlor, zu verstehen.

Die Verbindungen der Formel I können analog an sich bekannter Verfahren hergestellt werden (vgl. u.a. die deutschen Offenlegungsschriften Nr. 2.123.236, 2.601.780, und 3.240.975).

So kann man z.B. eine Verbindung der Formel I dadurch erhalten, dass man,

a) eine Verbindung der Formel II

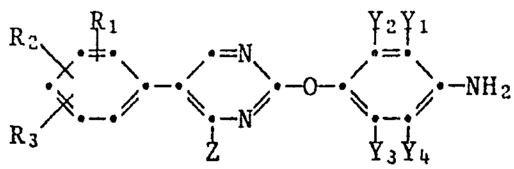

(II)

mit einer Verbindung der Formel III

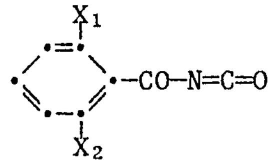

(III)

oder

b) eine Verbindung der Formel IV

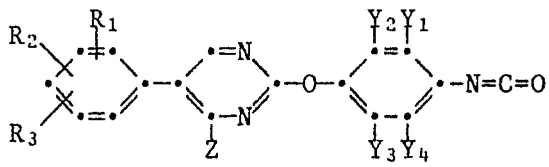

(IV)

mit einer Verbindung der Formel V

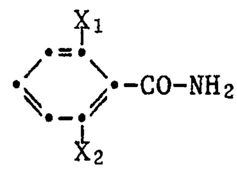

(V)

oder

c) eine Verbindung der Formel II mit einer Verbindung der Formel VI

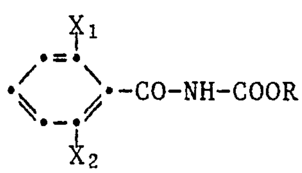

(VI)

umsetzt, wobei in den Formeln II bis VI die Reste $X_1$, $X_2$, $Y_1$ bis $Y_4$, Z und $R_1$ bis $R_3$ die vorstehend angegebenen Bedeutungen haben und R für einen $C_1$-$C_8$-Alkylrest, der gegebenenfalls mit Halogen, vorzugsweise Chlor, substituiert ist, steht.

Die erwähnten Verfahren a), b) und c) werden vorzugsweise unter normalem Druck und in Gegenwart eines organischen Lösungs-oder Verdünnungsmittels durchgeführt. Als Lösungs-oder Verdünnungsmittel eignen sich z.B. Aether und ätherartige Verbindungen, wie Diäthyläther, Dipropyläther, Dibutyläther, Dioxan, Dimethoxyäthan und Tetrahydrofuran; N,N-dialkylierte Carbonsäureamide; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol; Nitrile, wie Acetonitril oder Propionitril; Dimethylsulfoxid sowie Ketone, z.B. Aceton, Metyläthylketon, Methylisopropylketon und Methylisobutylketon. Verfahren a) wird im allgemeinen bei einer Temperatur von -10 bis 200°C, vorzugsweise zwischen 30 und 150°C, gegebenenfalls in Gegenwart einer organischen Base, z.B. Triäthylamin, durchgeführt. Die Durchführung von Verfahren b) erfolgt bei einer Temperatur von 0 bis 150°C, vorzugsweise beim Siedepunkt des verwendeten Lösungsmittels, und gegebenenfalls in Gegenwart einer organischen Base, wie Pyridin, und/oder unter Zusatz eines Alkali-oder Erdalkalimetalls, vorzugsweise Natrium. Für das Verfahren c), d.h. für die Umsetzung der Urethane der Formel VI mit einem Pyridyloxyanilin der Formel II, werden Temperaturen zwischen etwa 60°C und dem Siedepunkt des jeweiligen Reaktionsgemisches bevorzugt, wobei als Lösungsmittel insbesondere aromatische Kohlenwasserstoffe, wie Toluol, Xylole, Chlorbenzol usw., verwendet werden.

Die Ausgangsstoffe der Formeln III, V und VI sind bekannt oder sie können analog bekannten Verfahren hergestellt werden. Bei den Ausgangsstoffen der Formel II un IV handelt es sich um neuartige Verbindungen, die ebenfalls einen Gegenstand der vorliegenden Erfindung bilden. Man kann die 5-Phenylpyrimid-2-yloxy-aniline der Formel II herstellen, indem man ein in 2-Stellung umsetzungsfähiges 5-Phenylpyrimidin der Formel VII

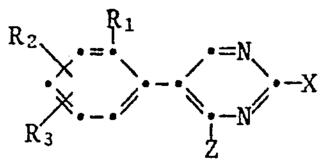

(VII)

mit einem p-Aminophenol der Formel VIII

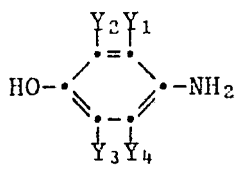

(VIII)

umsetzt, wobei in den Formeln VII und VIII die Reste $Y_1$ bis $Y_4$, Z und $R_1$ bis $R_3$ die vorstehend angegebenen Bedeutungen haben und X Halogen, vorzugsweise Chlor, oder $-SO_2CH_3$ bedeutet.

Die substituierten 5-Phenylpyrimidine der Formel VII sind wie folgt in an sich bekannter Weise zugänglich [Vgl. Arch. Pharmaz. Ber. dtsch. pharmaz. Ges. 317, 425 (1984)]:

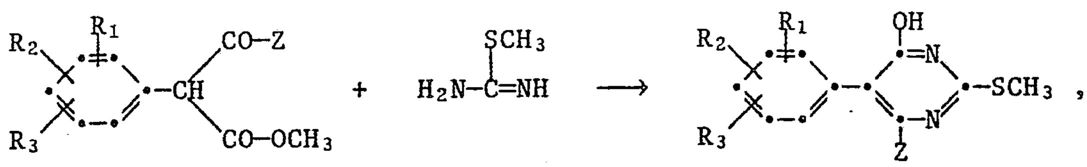

wobei in den obigen Formeln $R_1$ bis $R_3$ und Z die vorstehend angegebenen Bedeutungen haben. Aus dem so entstandenen 2-Methylthio-4-hydroxy-5-phenylpyrimidin wird durch Halogenierung mit Phosphoroxychlorid ($POCl_3$) und nachfolgende katalytische Hydrierung die 4-Hydroxygruppe entfernt [vgl. Rec. 92 1025 (1973); DE-Patentschrift Nr. 3,423,622]. Das gebildete 2-Metylthio-5-phenylpyrimidin der Formel (X)

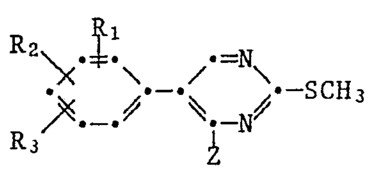

(X)

kann dann durch Oxydation, z.B. mit p-Cl-Perbenzoesäure [vgl. J. Chem. Soc. C 568 (1967)], in eine Verbindung der Formel VII übergeführt werden, worin X Methylsulfonyl bedeutet.

Für die Herstellung von Verbindungen der Formel X bzw. VII, worin Z Trihalogenmethyl, vorzugsweise $-CF_3$ und $-CF_2Cl$, oder Pentafluoräthyl bedeutet, wird erfindungsgemäss ein neuartiges Verfahren vorgeschlagen, bei dem z.B. Trifluormetylcarbonylmethoxystyrol, der Formel IX mit S-Methylisothioharnstoff umgesetzt wird

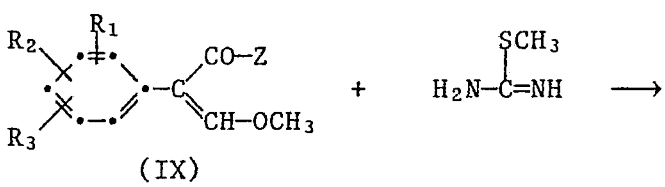

(X)

(IX)

Die erhaltene Verbindung der Formel X wird dann wie oben angegeben in eine entsprechende 2-Methylsulfonylverbindung der Formel VII überführt:

(X)

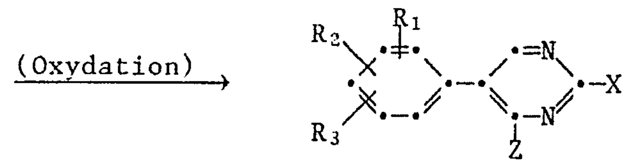


(VII)

X = $-SO_2CH_3$
Z =Trihalogenmethyl, $-CF_2CF_3$

Ein Trihalogenmetylcarbonyl-oder Pentafluoräthylcarbonylmethoxystyrol der vorstehenden Formel IX kann hergestellt werden durch Acylierung des entsprechenden Methoxystyrols, das vorzugsweise am Benzolring halogensubstituiert ist, mit entsprechenden halogenierten Säureanhydriden:

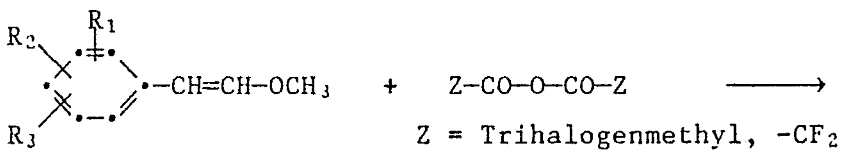

(IX)

Z = Trihalogenmethyl, $-CF_2CF_3$

Diese Umsetzung kann in einem Lösungsmittel, z.B. Pyridin, oder ohne Lösungsmittel im Bombenrohr (4 bis 10 Stunden bei etwa 80 bis 120°C) vorgenommen werden.

Verbindungen der Formel VII, worin X Halogen, vorzugsweise Chlor, bedeutet, sind erhältlich indem man ein entsprechendes Metholystyrolderivat der Formel IX in Gegenwart einer Base mit Guanidin, vorzugsweise in Form eine Salzes, z.B. des Guanidinhydrochlorids, umsetzt und das gebildete 2-Aminopyrimidin der Formel XI in üblicher Weise diazotiert und in ein 2-Halogenpyrimidin der Formel VII überführt

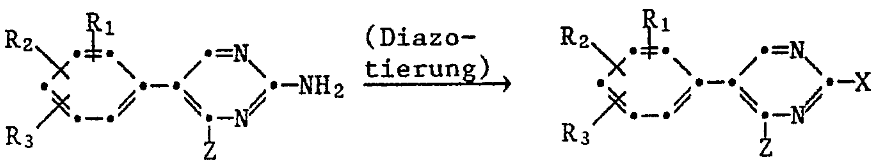


(XI) (VII)

X = Halogen
Z = Trihalogenmethyl, $-C_2F_5$

Ein 5-Phenylpyrimidin der Formel VII, worin X Halogen, vorzugsweise Chlor, und Z Methyl bedeutet, kann aus einem gemäss dem in der DE-Patentschrift Nr. 3.315.797 beschriebenen Verfahren erhältlichen 2-Hydroxy-4-methyl-5-phenylpyrimidin der nachstehenden Formel durch Halogenierung, z.B. mit $POCl_3$, hergestellt werden:

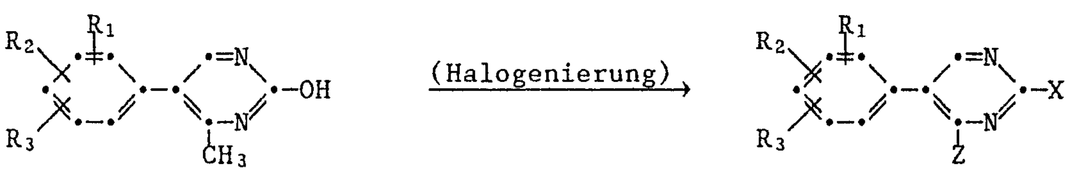


(VII) X = Halogen
Z = $CH_3$

In den obigen Formeln haben $R_1$ bis $R_3$ die vorstehend unter Formel I angegebenen Bedeutungen.

Zu Benzoylisocyanaten gemäss Formel III kann man unter anderem wie folgt gelangen (vgl. J. Agr. Food Chem. 21, 348 und 993; 1973):

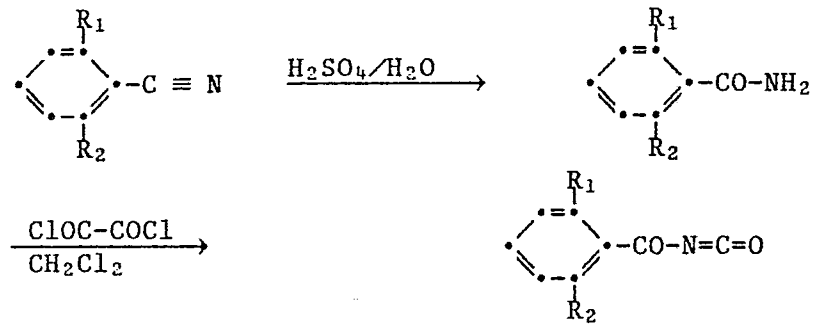


(III)

Die substituierten 4-Pyrimidinyloxy-phenylisocyanate der Formel IV lassen sich z.B. durch Phosgenierung der entsprechenden 4-Pyrimidinyloxy-aniline der Formel II nach allgemein üblichen Verfahren herstellen (vgl. DE-Patentschrift Nr. 3,326,509). Die weiterhin als Ausgangsstoffe zu verwendenden Benzamide der Formel V sind bekannt (vgl. z.B. Beilstein "Handbuch der organischen Chemie" Bd. 9, S. 336).

Die Urethane der Formel VI können in an sich bekannter Weise erhalten werden durch Umsetzung eines Benzoylisocyanats der Formel III mit einem entsprechenden Alkohol. Urethane der Formel VI sind ferner herstellbar durch Umsetzung eines Benzamides der Formel V in Anwesenheit einer basischen Verbindung mit einem entsprechenden Ester der Chlorameisensäure.

Aus der deutschen Offenlegungsschrift Nr. 3311703 sind bereits N-Benzoyl-N'-pyramidyloxyphenylharnstoffe als Insektizide bekannt, in denen die Phenylgruppe einen Pyrimidin-5-yloxy-Rest aufweist; weiterhin werden in der japanischen Patentpublikation 56015-272 insektizide N-Benzyol-N'-pyrimidin-2-yloxyphenyl-harnstoffe erwähnt, deren Pyrimidinylrest in 5-Stellung mit Halogen oder Trifluormethyl, nicht aber mit einem aromatischen Rest, substituiert ist. Die erfindungsgemässen Verbindungen der Formel I unterscheiden sich strukturell demgegenüber im wesentlichen dadurch, dass ihr Phenylrest mit einer Pyrimidin-2-yloxygruppe, die ihrerseits in 5-Stellung eine Phenylgruppe trägt, substituiert ist.

Ueberraschenderweise wurde nun gefunden, dass sich die vorliegenden Verbindungen der Formel I bei guter Dauerwirkung und Pflanzenverträglichkeit, hoher Nützlingsschonung und geringer Warmblütertoxizität ausgezeichnet als Schädlingsbekämpfungsmittel eignen. Sie zeigen besondere Wirksamkeit vor allem bei der Bekämpfung von Pflanzen und Tiere befallenden Vertretern der Ordnung Akarina und von Insekten.

Insbesondere eignen sich die Verbindungen der Formel I zur Bekämpfung von Insekten der Ordnungen: Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenotera, sowie speziell von Vertretern der Ordnung Akarina der Familien: Ixodidae, Argasidae, Tetranychidae und Dermanyssidae.

Neben ihrer Wirkung gegenüber Fliegen, wie z.B. Musca domestica, und Mückenlarven können Verbindungen der Formel I auch zur Bekämpfung von pflanzenschädigenden Frassinsekten in Zier-und Nutzpflanzungen, insbesondere in Baumwollkulturen (z.B. Spodoptera littoralis und Heliothis virescens), sowie in Obst-und Gemüsekulturen (z.B. Las peyresia pomonella, Leptinotarsa decemlineata und Epilachna varivestis) eingesetzt werden. Die Verbindungen der Formel I zeichnen sich durch eine ausgeprägte larvizide und ovo-larvizide Wirkung gegen Insekten, insbesondere Larven von fressenden Schadinsekten, aus. Werden Verbindungen der Formel I von adulten Insekten-Stadien mit den Futter aufgenommen, so ist in vielen Fällen, insbesondere bei Coleopteren, wie z.B. Anthonomus grandis, eine verminderte Ei-Ablage und/oder reduzierte Schlupfrate festzustellen.

Die Verbindungen der Formel I können ferner zur Bekämpfung von Ektoparasiten, wie Lucilia sericata, an Haus-und Nutztieren eingesetzt werden, z.B. durch Tier-, Stall-und Weidebehandlung.

Die erfindungsgemässen Verbindungen der Formeln I weisen eine besonders ausgeprägte Wirkung gegen pflanzenschädigende Akariden (Spinnmilben: z.B. der Familien Tetranychidae, Tarsonemidae, Eriophydae, Tyroglyphidae und Glycyphagidae) und auch gegen ektoparasitäre Akariden (Milben und Zecken: z.B. der Familien Ixodidae, Argasidae, Sarcoptidae und Dermanyssidae), welche Nutztiere befallen, auf. Einige der erfindungsgemässen Verbindungen zeichnen sich durch gute akarizid-ovizide Aktivität und Blattpenetrationswirkung aus. Die erfindungsgemässen Verbindungen eigenen sich vor allem zur Bekämpfung der folgenden, Obst-und Gemüsekulturen befallenden Milbenspezies: Tetranychus urticae, Tetranychus cinnabarinus, Panonychus ulmi, Bryobia rubrioculus, Panonychuis citri, Eriophyes pyri, Eriophyes ribis, Eriophyes vitis, Tarsomemus pallidus, Phyllocoptes vitis und Phyllocopture oleivora.

Die gute pestizide Wirkung der erfindungsgemässen Verbindungen der Formel I entspricht einer Abtötungsrate (Mortalität) von mindestens 50 - 60 % der erwähnten Schädlinge.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoff klassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und können daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formel I, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl-oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl-oder -äthyl-äther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymierisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier-und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali-oder gegebenenfalls substituierten Ammoniumsalze von höheren Fettsäuren ($C_{10}$-$C_{22}$), wie z.B. die Na-oder K-Salze der Oel-oder Stearinsäure, oder von natürlichen Fettsäuregemischen, die z.B. aus Kokosnuss-oder Tallöl gewonnen werden können. Ferner sind als Tenside auch die Fettsäure-methyltaurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali-oder gegebenefalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na-oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8 - 22 C-Atomen. Alkylarlysulfonate sind z.B. die Na-, Ca-oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome in Alkylrest der Alkylphenole enthalten können. Weiterhin geeignete nichtionische

Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyathylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl-oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:
"Mc Cutcheon's Detergents and Emulsifiers Annual"
MC Publishing Corp., Ridgewood, New Jersey, 1979;
Dr. Helmut Stache "Tensid Taschenbuch", Carl Hanser Verlag München/Wien 1981.

Die pestiziden Zubereitungen enthalten in der Regel 0,1 bis 99 %, insbesondere 0,1 bis 95 %, Wirkstoff der Formel I oder Kombinationen dieser Wirkstoffe mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wersentlich geringere Wirkstoffkonzentrationen aufweisen, wie z.B. 0,1 bis 1000 ppm.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1: Herstellung von Ausgangs-und Zwischenprodukten

a) Herstellung von 1.1.1-Trifluor-3-phenyl-4-methoxy-3-buten-2-on:

Bei -10° bis 0°C werden in einer Argonatmosphäre 201 ml Trifluoressigsäureanhydrid langsam zu einer Lösung von 150 g $\beta$-Methoxystyrol und 27,4 ml Pyridin in 450 ml Methylenchlorid getropft. Anschliessend wird das Reaktionsgemische ohne Kühlung ausgerührt und danach 17 Stunden am Rückfluss erhitzt. Nach dem Abkühlen wird die Lösung auf 600 ml Eiswasser gegossen. Die organische Phase wird abgetrennt und nochmals mit Wasser, 1N Natriumbicarbonat-Lösung und dann mit gesättigter Kochsalzlösung gewaschen. Nach dem Trocknen über Magnesiumsulfat wird das Lösungsmittel am Rotationsverdampfer abgetrieben und die so erhaltene rote Flüssigkeit in Hochvakuum destilliert. Man erhält so die Titelverbindung der Formel

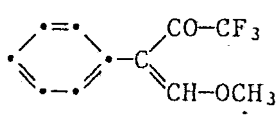

mit einem Sdp. von 81 - 84°C/$10^{-2}$mm.

b) Herstellung von 2-Methylthio-4-trifluormethyl-5-phenyl-pyrimidin:

Es werden 46 g 1.1.1-Trifluor-3-phenyl-4-methoxy-3-buten-4-on in ein Gemisch aus 150 ml 30 %-iger, wässriger Kaliumcarbonatlösung, 200 ml Aethanol und 28 g S-Methylisothioharnstoff-sulfat eingetropft. Anschliessend wird das Reaktionsgemisch während 34 Stunden am Rückfluss erhitzt. Nach dem Abdestillieren des Aethanols wird der Rückstand mit Eiswasser versetzt und mit Aether extrahiert. Das Lösungsmittel wird abdestilliert und die zurückbleibende rote Flüssigkeit durch Säulenchromatographie gereinigt.

Man erhält auf diese Weise die Titelverbindung der Formel

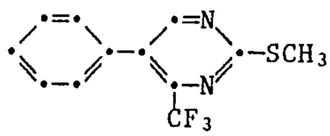

mit einem Brechungsindex $n_D^{20}$ = 1,5716.

c)Herstellung von 2-Methylsulfonyl-4-trifluormethyl-5-phenyl-pyrimidin:

Zu einer auf 5°C gekühlten Lösung von 18,5 g 2-Methylthio-4-trifluormethyl-5-phenyl-pyrimidin in 500 ml Chloroform tropft man im Verlauf von 2 Stunden langsam und unter Rühren eine Lösung von 26 g 40 %iger Peressigsäure in 20 ml Chloroform, so dass die Temperatur nicht über 10°C steigt. Anschliessend wird noch während 70 Stunden bei Raumtemperatur nachgerührt, bis kein Ausgangsmaterial mehr im Dünnschichtchromatogramm nachweisbar ist. Die filtrierte Lösung wird dann im Vakuum zur Trockene eingedampft. Man erhält blassgelbe Kristalle der Titelverbindung der Formel

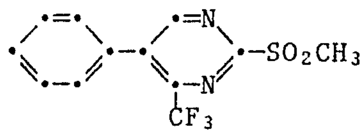

vom Smp. 110 - 112°C.

d) Herstellung von 4-(4-Trifluormethyl-5-phenyl-pyrimidin-2-yloxy)-anilin :

Zu einer Lösung von 2,1 g 4-Aminophenol in 50 ml Dimethylsulfoxid gibt man unter Kühlung 2,2 g gepulvertes Kaliumhydroxid und rührt während 1 Stunde, wonach sich eine klare Lösung bildet. Zu dem Ansatz gibt man portionsweise 6 g 2-Methylsulfonyl-4-trifluormethyl-5-phenyl-pyrimidin und rührt die Mischung nochmals während 4 Stunden bei Raumtemperatur. Man giesst das Gemisch auf Eiswasser und extrahiert mit Aether: Die gewaschenen und getrockneten Aetherextrakte ergeben ein der Titelverbindung der Formel

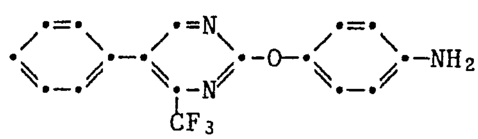

entsprechendes Produkt vom Smp.123 - 126°C.

Beispiel 2: Herstellung von N-(2,6-Difluorbenzoyl)-N'-[ (5-phenyl-4-trifluormethyl-pyrimid-2-yloxy)-phenyl]-harnstoff:

Zu einer auf 60°C erwärmten, und unter Stickstoff gerührten Lösung von 6 g 4-(4-Trifluormethyl-5-phenyl-pyrimidin-2-yloxy)-anilin in 100 ml trockenem Toluol tropft man eine Lösung von 3 g 2.6-Difluorbenzoylisocyanat in 15 ml Toluol ein. Nach Abklingen der exothermen Reaktion rührt man noch während 1 Stunde bei 80°C. Nach dem Abkühlen der orangefarbenen Reaktionslösung wird diese mit 50 ml Hexan verdünnt und das ausgefallene Produkt abgesaugt. Nach dem Waschen mit Aether und Trocknen erhält man 5,5 g eines beige-farbenen Kristallpulvers vom Smp. 197 - 199°C, das der Titelverbindung der Formel

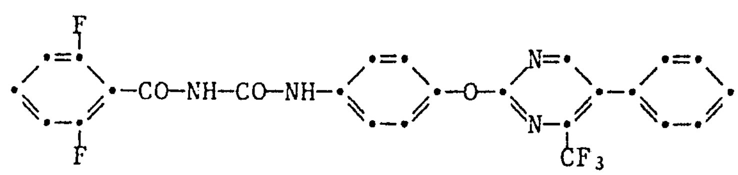

(Verbindung Nr. 1) entspricht.

Wie vorstehend beschrieben werden weiterhin die folgenden Verbindungen der Formel I hergestellt:

| Verb. Nr. | $X_1$ | $X_2$ | $Y_1$ | $Y_2$ | $Y_3$ | $Y_4$ | Z | $R_1$ | $R_2$ | $R_3$ | Smp.[°C] |
|---|---|---|---|---|---|---|---|---|---|---|---|
| 2 | Cl | Cl | H | H | H | H | $-CF_3$ | H | H | H | >105° (Zers.) |
| 3 | F | F | H | Cl | H | H | $-CF_3$ | H | H | H | 188-191° |
| 4 | F | F | H | Cl | Cl | H | $-CF_3$ | H | H | H | 181-4° |
| 5 | F | F | $-CH_3$ | H | H | H | $-CF_3$ | H | H | H | 183-6° |
| 6 | $-OCH_3$ | F | H | H | H | H | $-CF_3$ | H | H | H | 154-8° |
| 7 | Cl | H | H | Cl | Cl | H | $-CF_3$ | H | H | H | 234-7° |
| 8 | $-SCH_3$ | F | H | H | H | H | $-CF_3$ | H | H | H | 138-141° |
| 9 | Cl | H | H | Cl | H | H | $-CF_3$ | H | H | H | 201-4° |
| 10 | F | F | H | H | H | H | $-CF_2Cl$ | H | H | H | 196-7° |
| 11 | Cl | H | H | H | H | H | $-CF_2Cl$ | H | H | H | 186-7° |
| 12 | Cl | H | H | H | H | H | $-CF_3$ | H | H | H | 195-7° |
| 13 | F | F | H | H | H | H | $-CH_3$ | H | H | H | 199-200° |
| 14 | Cl | H | H | H | H | H | $-CH_3$ | H | H | H | 171-3° |
| 15 | F | F | H | $-CH_3$ | H | H | $-CF_3$ | H | H | H | 186-8° |
| 16 | F | F | H | H | H | H | $-CF_3$ | 4-Cl | H | H | 182-4° |
| 17 | F | F | H | Cl | H | Cl | $-CF_3$ | H | H | H | amorphe Masse |
| 18 | F | H | H | Cl | H | Cl | $-CF_3$ | H | H | H | 195-7° |
| 19 | F | F | $-CH_3$ | H | H | $-CH_3$ | $-CF_3$ | H | H | H | 174-7° |
| 20 | Cl | Cl | $-CH_3$ | H | H | $-CH_3$ | $-CF_3$ | H | H | H | 184-8° |
| 21 | F | F | H | H | H | H | $-CF_3$ | 2-F | H | H | 175-7° |
| 22 | Cl | Cl | H | H | H | H | $-CF_3$ | 2-F | H | H | 215-7° |
| 23 | F | F | $-CH_3$ | H | H | H | $-CF_3$ | 2-F | H | H | 161-3° |
| 24 | F | F | H | H | H | H | $-CF_3$ | 2-Cl | H | H | 194-5° |
| 25 | F | F | $-CH_3$ | H | H | H | $-CF_3$ | 3-Cl | H | H | 190-2° |
| 26 | F | F | H | H | H | H | $-CF_3$ | 3-Cl | H | H | 168-170° |
| 27 | F | F | $-CH_3$ | H | H | H | $-CF_3$ | 2-Cl | H | H | 197-199° |
| 28 | Cl | Cl | $-CH_3$ | H | H | H | $-CF_3$ | 2-F | H | H | 105-6° |
| 29 | Cl | Cl | $-CH_3$ | H | H | H | $-CF_3$ | 2-Cl | H | H | 156-9° |
| 30 | F | F | $-CH_3$ | H | H | H | $-CF_2Cl$ | 2-Cl | H | H | 177-9° |
| 31 | F | F | F | H | H | H | $-CF_3$ | H | H | H | 192-3° |
| 32 | F | F | $-CF_3$ | H | H | H | $-CF_3$ | H | H | H | 158-160° |
| 33 | F | F | H | H | H | H | $-CF_2CF_3$ | H | H | H | 178-180° |
| 34 | Cl | Cl | H | H | H | H | $-CF_2CF_3$ | H | H | H | 203-205° |

Beispiel 3:

Formulierungen für Wirkstoffe der Formel 1 gemäss Beispiel 2 resp. Kombinationen dieser Wirkstoffe mit andern Insektiziden oder Akariziden (% = Gewichtsprozent)

| 3.1 Spritzpulver | a) | b) | c) |
|---|---|---|---|
| Wirkstoff oder Wirkstoffkombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

3.2 Emulsions-Konzentrat

Wirkstoff oder Wirkstoffkombination 10 %
Octylphenolpolyäthylenglykoläther (4 - 5 Mol AeO) 3 %
Ca-Dodecylbenzolsulfonat 3 %
Ricinusölpolyglykoläther (36 Mol AeO) 4 %
Cyclohexanon 30 %
Xylolgemisch 50 %

Aus diesem Konzentrat können durch Verdünnen mit Wasser Emulsionen jeder gewünschten Konzentration hergestellt werden.

| 3.3 Stäubemittel | a) | b) |
|---|---|---|
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff oder die Wirkstoffkombination mit dem Träger vermischt und auf einer geeigneten Mühle vermahlen wird.

3.4 Extruder-Granulat

Wirkstoff oder Wirkstoffkombination 10 %
Na-Ligninsulfonat 2 %
Carboxymethylcellulose 1 % Kaolin 87 %

Der Wirkstoff oder die Wirkstoffkombination wird mit den Zusatzstoffen vermischt, vermahlen und mit Wasser angefeuchtet. Dieses Gemisch wird extrudiert, granuliert und anschliessend im Luftstrom getrocknet.

3.5 Umhüllungs-Granulat

Wirkstoff oder Wirkstoffkombination 3 %
Polyäthylenglykol (MG 200) 3 %
Kaolin 94 %

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

3.6 Suspensions-Konzentrat

Wirkstoff oder Wirkstoffkombination 40 %
Aethylenglykol 10 %
Nonylphenolpolyäthylenglykoläther (15 Mol AeO) 6 %
Na-Ligninsulfonat 10 %
Carboxymethylcellulose 1 %
37%ige wässrige Formaldehyd-Lösung 0,2 %
Silikonöl in Form einer 75%igen wässrigen Emulsion 0,8 %
Wasser 32 %

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 4: Wirkung gegen Musca domestica:

Je 50 g frisch zubereitetes Nährsubstrat für Maden wird in Becher eingewogen. Von einer 1 Gew.%igen acetonischen Lösung des betreffenden Wirkstoffes wird eine bestimmte Menge auf das in den Bechern befindliche Nährsubstrat pipettiert, so dass sich eine Wirkstoffkonzentration von 800 ppm ergibt. Nach dem Durchmischen des Substrates lässt man das Aceton mindestens 20 Stunden lang verdampfen.

Dann werden pro Wirkstoff und Konzentration je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert. Die pro Ansatz ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Verbindungen der Formel I gemäss Beispiel 2 zeigen gute Wirkung im obigen Test.

Beispiel 5: Wirkung gegen Tetranychus urticae (OP-sensible) und Tetranychus cinnabarinus (OP-tolerant).

Die Primärblätter von Phaseolus volgaris-Pflanzen wurden 12 Stunden vor dem Versuch auf akarizide Wirkung mit einem infestierten Blattstück aus einer Massenzucht von Tetranychus urticae (OP-sens.) oder Tetranychus cinnabarinus (OP-tol.) belegt (Mischpopulation). Die Toleranz bezieht sich auf die Verträglichkeit gegenüber Diazinon.

Die so behandelten infestierten Pflanzen werden mit einer Versuchslösung in Emulsionsform enthaltend 400 ppm der zu prüfenden Verbindung bis zur Tropfnässe besprüht.

Nach 24 Stunden und wiederum nach 6 (T.urticae) bzw. 7 Tagen (T.cinnabarinus) werden Imagines und Larven (alle beweglichen Stadien) sowie abgelegte Eier unter dem Binokular auf lebende und tote Individuen ausgewertet.

Man verwendet pro Testspezies eine Pflanze. Während des Versuchsverlaufs stehen die Pflanzen in Gewächshauskabinen bei 25°C mit etwa 50 - 60 % rel. Luftfeuchtigkeit.

Die Verbindung Nr. 1 gemäss Beispiel 2 zeigt in diesem Versuch 90 - 100 %-ige Wirkung (Mortalität) gegen Tetranychus urticae und gute Wirksamkeit gegen Tetranychus cinnabarinus.

Beispiel 6: Ovizide Wirkung auf Tetranychus urticae (OP-resistent)

Eingetopfte Phaseolus vulgaris-Pflanzen im Primärblatt-Stadium werden jeweils zweimal mit 30 Weibchen von Tetranychus urticae besiedelt. Nach 24-stündiger Eiablage werden die Weibchen mit einer Saugpumpe (waserstrahlpumpe) von den Pflanzen entfernt, so dass auf diesen nur noch die abgelegten Milbeneier verbleiben.

Die mit den Milbeneiern infestierten Pflanzen werden dann mit einer wässrigen Emulsion enthaltend 400 ppm der zu prüfenden Verbindung bis zur Tropfnässe besprüht und 5 Tage bei 25°C und etwa 50 % relativer Luftfeuchtigkeit gehalten. Nach dieser Zeit wird die Prozentuale Mortalität der Eier und geschlüpften Larven durch Auszählen bestimmt.

Verbindungen der Formel I gemäss dem vorstehenden Beispiel 2 zeigen gute Wirkung in obigem Test.

Beispiel 7: Pflanzenmitizide Blattpenetrations-Wirkung auf Tetranychus cinnabarinus

Für den Versuch werden eingetopfte Buschbohnenpflanzen im Primärblatt-Stadium, die mit Tetranychus cinnabarinus infestiert sind, verwendet. Die Besiedlung mit den Milben erfolgt 1 Tag vor der Wirkstoffapplikation.

Die Blattoberseiten der mit diesen Milben infestierten Versuchspflanzen werden mit einer Emulsionszubereitung enthaltend 400 ppm des zu prüfenden Wirkstoffes besprüht. Nach dem Antrocknen des Sprühbelages wird jeweils der Rand der Blattoberseite einer Anzahl befallener Blätter mit einem Wulst aus zähflüssigem Leim (Raupenleim) abgegrenzt, um ein Ueberlaufen der Milben von der Blattunterseite auf die Blattoberseite zu verhindern.

Die behandelten Pflanzen werden dann im Gewächshaus bei einer Temperatur von 25°C bis 27°C und einer relativen Luftfeuchtigkeit von ca. 50 % gehalten. Sechs Tage nach der Wirkstoffapplikation wird festgestellt, ob eine translaminare Wirkung, d.h. Blattpenetration des Wirkstoffs von der Blattoberseite zur Blattunterseite, eingetreten war, und zwar durch Bestimmung der prozentualen Mortalität der Eier und larvalen sowie adulten Stadien.

Verbindungen der Formel I gemäss dem vorstehenden Beispiel 2 zeigen gute Wirkung in obigem Test.

Beispiel 8: Wirkung gegen Panonychus ulmi (OP und Carb. resistent)

Eingetopfte Apfelsämlinge mit etwa 20 - 30 Blättern werden mit jeweils 60 adulten Weibchen von Panonychus ulmi besiedelt. Nach sieben Tagen werden die infestierten Pflanzen mit einer wässrigen Emulsion enthaltend 400 ppm der zu prüfenden Verbindung bis zur Tropfnässe besprüht. Die behandelten Pflanzen werden dann während weiterer 14 Tage bei 25°C und etwa 50 % relativer Luftfeuchtigkeit im Gewächshaus aufgestellt.

Nach dieser Zeit erfolgt die Auswertung, indem man 20 Blätter pro Pflanze entnimmt, die Milbenpopulation mittels einer Abbürst-Vorrichtung von den entnommenen Blättern entfernt und unter dem Binocular nach Eiern, postembryonalen Stadien und Adulten auszählt. Bewertet wird die prozentuale Reduktion der Milbenpopulation gegenüber unbehandelten Kontrollen.

Verbindungen der Formel I gemäss dem vorstehenden Beispiel 2 zeigen gute Wirkung im obigen Test.

Beispiel 9: Wirkung gegen tierparasitäre Milben

Von mit Dermanyssus gallinae befallenen Hühnern werden Ansätze bestehend aus etwa 50 Milben verschiedener Stadien (Larven, Nymphen und Imagines) entnommen. Die Milbenansätze werden jeweils mit einer wässrigen Emulsion, Suspension bzw. Lösung enthaltend 800 ppm des zu prüfenden Wirkstoffes benetzt. Hierzu werden die Milbenansatze in einem Teströhrchen mit der den Wirkstoff enthaltenden flüssigen Zubereitung übergossen; die Flüssigkeit wird anschliessend mit Hilfe eines Wattebausches aufgesogen. Die so behandelten Milben verbleiben während 72 Stunden in dem Teströhrchen. Nach dieser Zeit wird die prozentuale Abtötung der behandelten Milben gegenüber unbehandelten Kontrollansätzen ermittelt.

Verbindungen der Formel I gemäss dem vorstehenden Beispiel 2 zeigen gute Wirkung im obigen Test.

Beispiel 10: Wirkung gegen Zecken: Abtötungswirkung in verschiedenen Entwicklungsstadien

Als Testobjekte werden Larven (jeweils ca. 50), Nymphen (jeweils ca. 25) oder Imagines (jeweils ca. 10) der Zeckenarten Rhipicephalus bursa, Amblyomma hebraeum und Boophilus microplus verwendet. Die Testtiere werden für kurze Zeit in wässrige Emulsionen der zu untersuchenden Substanzen mit einer Konzentration von 400 ppm getaucht. Die in Teströhrchen befindlichen Emulsionen werden dann mit Watte aufgenommen und die benetzten Testtiere in den so kontaminierten Röhrchen belassen. Die Auswertung (% - Mortalität) erfolgt für Larven nach 3 Tagen und für Nymphen und Imagines nach 14 Tagen.

Verbindungen der Formel I gemäss Beispiel 2 sind in diesem Test gut wirksam.

Beispiel 11: Wirkung gegen Zecken: Eiablagehemmung

Als Testtiere werden vollgesogene adulte Weibchen der Rinderzecke Boophilus microplus verwendet. Es werden je 10 Zecken eines OP-resistenten Stammes (z.B. vom Biarra-Stamm) und eines normal empfindlichen Stammes (z.B. vom Yeerongpilly-Stamm) behandelt. Die Zecken werden auf mit Doppelklebeband bezogenen Platten fixiert und dann entweder mit wässrigen Emulsionen bzw. mit Lösungen enthaltend 800 ppm der zu untersuchenden Verbindung benetzt oder mit einem mit diesen Flüssigkeiten getränkten Wattebausch in Berührung gebracht. Anschliessend werden sie in einem klimatisierten Raum bei konstanten Bedingungen aufbewahrt. Die Auswertung erfolgt nach drei Wochen. Es wird die prozentuale Hemmung der Ablage von fertilen Eiern gegenüber unbehandelten Kontrollen ermittelt.

Verbindungen der Formel I gemäss Beispiel 2 zeigen gute Wirkung in obigem Test.

Beispiel 12: Wirkung gegen Aëdes aegypti:

Auf die Oberfläche von 150 ml Wasser, das sich in einem Behälter befindet, wird so viel einer 0,1 Gew.-%igen acetonischen Lösung des Wirkstoffes pipettiert, dass eine Konzentration von 800 ppm erhalten wird. Nach Verdunsten des Acetons wird der Behälter mit 30 bis 40 2-tägigen Aëdes-Larven beschickt. Nach 1, 2 und 5 Tagen wird die Mortalität geprüft.

Verbindungen der Formel I gemäss Beispiel 2 zeigen in diesem Test gute Wirkung gegen Aëdes aegypti.

Beispiel 13 : Insektizide Frassgift-Wirkung:

Ca. 25 cm hohe eingetopfte Baumwollpflanzen werden mit wässrigen Wirkstoffemulsionen besprüht, die den Wirkstoff jeweils in einer Konzentration von 400 ppm enthalten.

Nach dem Antrocknen des Sprühbelages werden die Baumwollpflanzen mit Spodoptera littoralis-bzw. Heliothis virescens-Larven im dritten larvalen Stadium besiedelt. Der Versuch wird bei 24°C und 60 % relativer Luftfeuchtigkeit durchgeführt. Nach 120 Stunden wird die %-Mortalität der Test-Insekten bestimmt.

Im obigen Test zeigen erfindungsgemässe Verbindungen der Formel I gemäss Beispiel 2 gute Wirksamkeit.

Beispiel 14: Wirkung auf Laspeyresia pomonella (Eier):

Abgelegte Eier von Laspeyresia pomonella, die nicht älter als 24 Stunden sind, werden auf Filterpapier für 1 Minute in eine acetonisch-wässrige Lösung, enthaltend 800 ppm des zu prüfenden Wirkstoffes, eingetaucht. Nach dem Antrocknen der Lösung werden die Eier in Petrischalen ausgelegt und bei einer Temperatur von 28°C belassen. Nach 6 Tagen wird der prozentuale Schlupf aus den behandelten Eiern bewertet und die %-Mortalität bestimmt.

Verbindungen der Formel I gemäss Beispiel 2 zeigen gute Wirkung in obigem Test.

Beispiel 15: Reproduktions-Beeinflussung von Anthonomus grandis:

Adulte Anthonomus grandis, die nach dem Schlupf nicht älter als 24 Stunden waren, werden in Gruppen zu jeweils 25 Käfern in Käfige mit Gitterwänden überführt. Die mit den Käfern besetzten Käfige werden sodann während 5 bis 10 Sekunden in eine acetonische Lösung, enthaltend 400 ppm des zu prüfenden Wirkstoffes, eingetaucht. Nachdem die Käfer wieder trocken sind, werden sie zur Kopulation und Eiablage in abgedeckte und Futter enthaltende Schalen eingesetzt. Abgelegte Eier werden zwei-bis dreimal wöchentlich mit fliessendem Wasser ausgeschwemmt, gezählt, durch zwei-bis dreistündiges Einlegen in ein wässriges Desinfektionsmittel desinfiziert und dann in Schalen, die eine geeignete Larvaldiät enthalten, deponiert. Nach 7 Tagen wird untersucht, ob sich aus den deponierten Eiern Larven entwickelt haben.

Zur Ermittlung der Dauer des die Reproduktion beeinflussenden Effektes der zu prüfenden Wirkstoffe wird die Eiablage der Käfer während eines Zeitraums von etwa 4 Wochen überprüft. Die Bonitierung erfolgt anhand der Verminderung der Anzahl abgelegter Eier und der daraus geschlüpften Larven im Vergleich zu unbehandelten Kontrollen.

Die Verbindungen der Formel I gemäss Beispiel 2 zeigen gute Wirkung im obigen Test.

**Ansprüche**

1. Verbindung der Formel I,

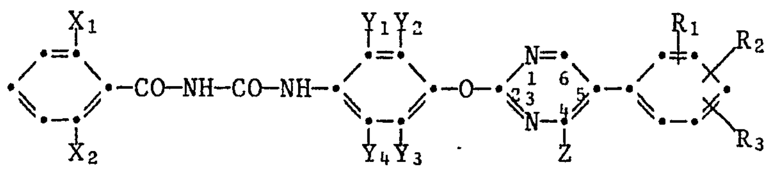

(I)

worin

$X_1$ Wasserstoff, Halogen, $C_1$-$C_3$-Alkoxy oder $C_1$-$C_3$-Alkylthio;
$X_2$ Halogen, Methyl, $C_1$-$C_3$-Alkoxy oder $C_1$-$C_3$-Alkylthio;
$Y_1$, $Y_2$, $Y_3$ und $Y_4$ unabhängig voneinander Wasserstoff, Halogen, Methyl, Trifluormethyl oder Methoxy;
Z Methyl, Halogenmethyl mit 1 bis 3 Halogenatomen oder Pentafluoräthyl;
R, $R_2$ und $R_3$ unabhängig voneinander Wasserstoff, Halogen, $C_1$-$C_3$-Alkyl; Trifluormethyl oder $C_1$-$C_3$-Alkoxy bedeuten.

2. Verbindung der Formel I gemäss Anspruch 1, dadurch gekennzeichnet, dass
$X_1$ Wasserstoff, Halogen, $C_1$-$C_3$-Alkoxy oder $C_1$-$C_3$-Alkylthio;
$X_2$ Halogen, Methyl, $C_1$-$C_3$-Alkoxy oder $C_1$-$C_3$-Alkylthio;
$Y_1$, $Y_2$, $Y_3$ und $Y_4$ unabhängig voneinander Wasserstoff, Halogen, Methyl, Trifluormethyl oder Methoxy;
Z Methyl, Halogenmethyl mit 1 bis 3 Halogenatomen;
R, $R_2$ und $R_3$ unabhängig voneinander Wasserstoff, Halogen, $C_1$-$C_3$-Alkyl, Trifluormethyl oder $C_1$-$C_3$-Alkoxy bedeuten.

3. Verbindung der Formel I gemäss Anspruch 1, dadurch gekennzeichnet, dass
$X_1$ Wasserstoff, Halogen oder Methoxy;
$X_2$ Halogen oder Methoxy;
$Y_1$ und $Y_4$ Wasserstoff;
$Y_2$ und $Y_3$ unabhängig voneinander Wasserstoff, Fluor, Chlor, Methyl oder Trifluormethyl;
2 Methyl, Dichlormethyl, Trichlormethyl, Monofluormethyl, Difluormethyl, Trifluormethyl, Difluorchlormethyl oder Pentafluoräthyl;
$R_1$, $R_2$ und $R_3$ unabhängig voneinander Wasserstoff, Fluor, Chlor, Brom, Methyl, Aethyl, i-Propyl, Trifluormethyl, Methoxy oder Aeithoxy bedeuten.

4. Verbindung der Formel I gemäss der Ansprüche 1 bis 3, dadurch gekennzeichnet, dass
$X_1$ Fluor, Chlor oder Methoxy und
$X_2$ Fluor oder Chlor bedeuten.

5. Verbindung der Formel I gemäss Ansprüche 1 bis 4, dadurch gekennzeichnet, dass $Y_1$ Methyl und $Y_2$, $Y_3$ und $Y_4$ Wasserstoff bedeuten.

6. Verbindung der Formel I gemäss Ansprüche 1 bis 4, dadurch gekennzeichnet, dass
$Y_1$ und $Y_4$ Wasserstoff und
$Y_2$ und $Y_3$ Wasserstoff, Fluor oder Chlor bedeuten.

7. Verbindung der Formel I gemäss Ansprüche 1 und 3 bis 6, dadurch gekennzeichnet, dass Z Trifluormethyl, Difluorchlormethyl oder Pentafluoräthyl bedeutet.

8. Verbindung der Formel I gemäss Ansprüche 1 bis 7, dadurch gekennzeichnet, dass
$R_1$ Wasserstoff,
$R_2$ Wasserstoff, 2-Fluor oder 2-Chlor und
$R_3$ Wasserstoff, 4-Fluor oder 4-Chlor bedeuten.

9. Verbindung gemäss einem der Ansprüche 1 bis 8, dadurch gekennzeichnet, dass $X_1$ und $X_2$ Fluor bedeuten.

10. Verbindung der Formel I gemäss einem der Ansprüche 1 bis 9, dadurch gekennzeichnet, dass $R_1$, $R_2$ und $R_3$ Wasserstoff bedeuten.

11. Verbindung gemäss Anspruch 7 der Formel

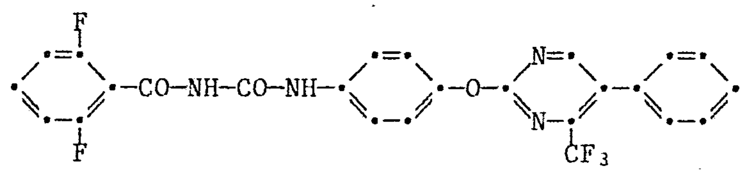
.

12. Verbindung gemäss Anspruch 7 der Formel

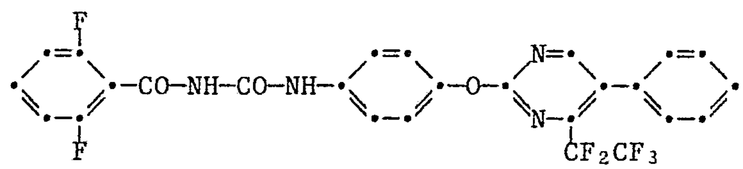
.

13. Verbindung gemäss Anspruch 7 der Formel

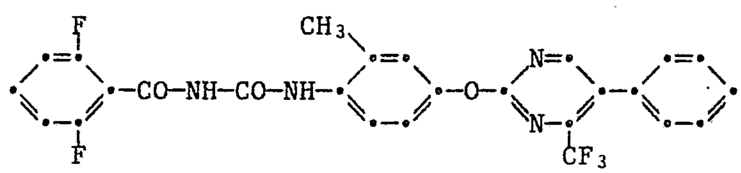
.

14. Verbindung gemäss Anspruch 7 der Formel

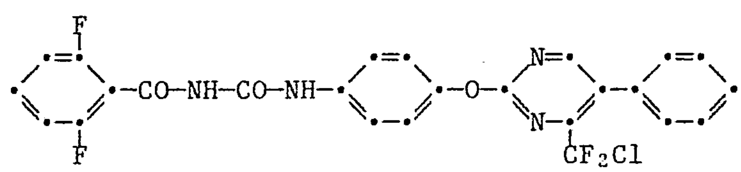
.

15. Verfahren zur Herstellung einer Verbindung der Formel I gemäss Anspruch 1 bis 14, dadurch gekennzeichnet, dass man

a) eine Verbindung der Formel II

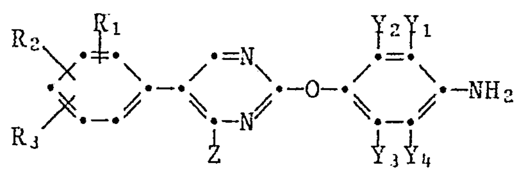 (II)

mit einer Verbindung der Formel III

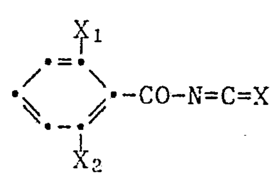 (III)

oder

b) eine Verbindung der Formel IV

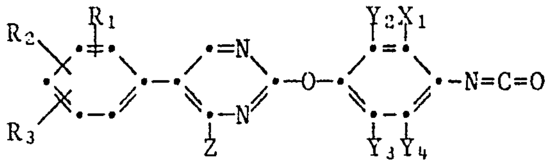

(IV)

mit einer Verbindung der Formel V

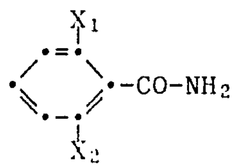

(V)

oder

c) eine Verbindung der Formel II mit einer Verbindung der Formel VI

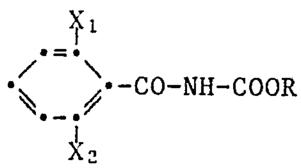

(VI)

umsetzt, wobei in den Formeln II bis VI die Reste $X_1$, $X_2$, $Y_1$ bis $Y_4$ und Z und $R_1$ bis $R_3$ die in den Ansprüchen 1 bis 10 angegebenen Bedeutungen haben und R für einen $C_1$-$C_8$-Alkylrest, der gegebenenfalls mit Halogen substituiert ist, steht.

16. Verbindung der Formel II gemäss Anspruch 15.

17. Verbindung der Formel IV gemäss Anspruch 15.

18. Verfahren zur Herstellung einer Verbindung der Formel II gemäss Anspruch 16, dadurch gekennzeichnet, dass man eine Verbindung der Formel VII

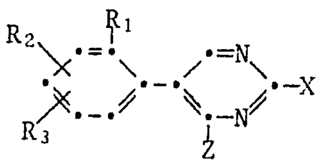

(VII)

mit einer Verbindung der Formel VIII

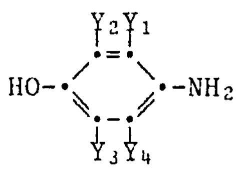

(VIII)

umsetzt, wobei in den Formeln VII und VIII die Reste $Y_1$ bis $Y_4$, Z und $R_1$ bis $R_3$ die in Anspruch 1 angegebenen Bedeutungen haben und X Halogen, vorzugsweise Chlor, oder Methylsulfonyl bedeutet.

19. Verfahren zur Herstellung einer Verbindung der Formel VII gemäss Anspruch 18, worin Z Halogenmethyl mit 1 bis 3 Halogenatomen oder Pentafluoräthyl und X Methylsulfonyl bedeuten, dadurch gekennzeichnet, dass man eine Verbindung der Formel IX

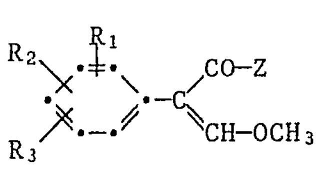

(IX)

S-Methylisothioharnstoff umsetzt und in der erhaltenen Verbindung der Formel X

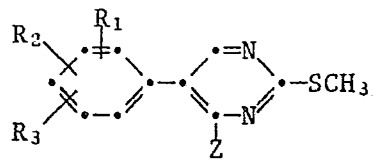

(X)

in an sich bekannter Weise die 2-Methylthiogruppe zur 2-Methylsulfonylgruppe oxydiert.

20. Verfahren gemäss Anspruch 19, dadurch gekenzeichnet, dass man eine Verbindung der Formel VII herstellt, worin Z Trifluormethyl, Difluorchlormethyl oder Pentafluoräthyl bedeutet.

21. Schädlingsbekämpfungsmittel, welches als aktive Komponente eine Verbindung gemäss Anspruch 1 bis 14, zusammen mit geeigneten Träger-und/oder anderen Zuschlagstoffen enthält.

22. Verwendung einer Verbindung gemäss Anspruch 1 bis 14 zur Bekämpfung von Schädlingen.

23. Verwendung gemäss Anspruch 22 zur Bekämpfung von Insekten und Vertretern der Ordnung Acarina.

24. Verwendung gemäss Anspruch 22 zur Bekämpfung von pflanzenschädigenden Akariden.

25. Verfahren zur Bekämpfung von Insekten und Vertretern der Ordnung Acarina, dadurch gekennzeichnet, dass man die Schädlinge bzw. deren verschiedene Entwicklungsstadien oder deren Aufenthaltsort mit einer pestizid wirksamen Menge einer Verbindung der Formel I gemäss einem der Ansprüche 1 bis 14 oder mit einem Mittel enthaltend neben Zusatz-und Trägerstoffen eine pestizid wirksame Menge dieser Verbindung, in Kontakt bringt oder behandelt.

Europäisches Patentamt

# EUROPÄISCHER RECHERCHENBERICHT

Nummer der Anmeldung

EP 87112241.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| A | EP - A2 - 0 123 861 (BAYER)<br>* Ansprüceh 1,5,6,7,8 *<br>-- | 1,15,<br>22,25,<br>26 | C 07 D 239/34<br>A 01 N 47/34 |
| A | US - B - 435 617 (JOHNSTON)<br>* Anspruch 1; Spalte 2, Zeile 64 - Spalte 7, Zeile 22 *<br>---- | 1,15 | |
| | | | RECHERCHIERTE SACHGEBIETE (Int. Cl.4) |
| | | | C 07 D 239/00 |

Der vorliegende Recherchenbericht wurde für alle Patentansprüche erstellt.

| Recherchenort | Abschlußdatum der Recherche | Prüfer |
|---|---|---|
| WIEN | 26-11-1987 | LUX |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82